# EUROPEAN PATENT APPLICATION

(11) **EP 4 005 561 A1**
(43) Date of publication of application: **01.06.2022**
(21) Application number: 20844154.3
(22) Date of filing: 26.07.2020
(51) Int. Cl.: A61K 9/19, A61K 9/107, A61K 47/60, A61K 47/10, A61K 31/352

(54) **NANO-MICELLE PREPARATION OF ICARITIN AND PREPARATION METHOD THEREFOR AND APPLICATION THEREOF**

(30) Priority: 25.07.2019 CN 201910679309
(71) Applicant: Beijing Shenogen Pharma Group Ltd., Beijing 102206 (CN)
(72) Inventor: TANG, Cheng, Beijing 102206 (CN); CHEN, Xiaoming, Beijing 102206 (CN); YAO, Hua, Beijing 102206 (CN); ZHANG, Ranran, Beijing 102206 (CN); MENG, Kun, Beijing 102206 (CN)
(74) Representative: Engelhard, Markus
(86) International application number: PCT/CN2020/104704
(87) International publication number: WO 2021/013262

(57) **Abstract**

A nano-micelle preparation of icaritin. The preparation comprises icaritin and polymer auxiliary materials. Further provided is a preparation method for the nano-micelle preparation, and the prepared nano-micelle preparation of icaritin has the advantage of high bioavailability.

## Description

### Technical Field

The present invention relates to a nano-micelle preparation of icaritin, a preparation method therefor and an application thereof, which belong to the field of medicines.

### Background Art

Icaritin, also known as anhydroicaritin, is a new effective monomer obtained by enzymatic conversion of icariin which is the main active ingredient extracted and isolated from the traditional Chinese medicine Epimedium, and has the structure represented by Formula (I):

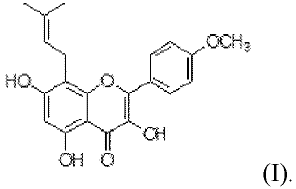

The preparation method for anhydroicaritin is disclosed in a Chinese patent with an application number of 200710099025.1, by which method icariin is enzymatically hydrolyzed with beta-glycosidase to obtain anhydroicaritin.

A Chinese patent with an application number of 200910025047.2 discloses an oral preparation of an anhydroicaritin liposome, the mass fractions of components of which preparation are: 50%-85% soy lecithin, 10-35% cholesterol, and 2-25% anhydroicaritin, wherein a phospholipid bilayer serves as the surface of the liposome, with a hydrophilic group outside the bilayer forming a hydrophilic cap, a hydrophilic group inside the bilayer constituting an interior aqueous phase, and a hydrophobic group between the bilayer constituting a hydrophobic region, and anhydroicaritin is embedded in the phospholipid bilayer of the liposome. However in practice, the above-mentioned preparation is costly and not suitable for large-scale production.

A Chinese patent with an application number of 200910184281.X discloses an anhydroicaritin-containing pharmaceutical composition, wherein the composition comprises anhydroicaritin and phospholipids with a mass ratio of 1 : 0.5-5. However, a study reveals a low bioavailability of the preparation.

A Chinese patent with an application number of 201410185323.2 discloses a crystal form of an icaritin compound, a drug containing the crystal form, and an application thereof. This patent additionally discloses an anhydrate crystal form of icaritin, the crystal form having the advantages of great stability, long shelf life, and being suitable for transportation and storage in extreme weather.

A Chinese patent with an application number of 201310526018.0 sets forth an oral preparation of icaritin, wherein the preparation comprises icaritin with a particle size below 90 µm, a vegetable oil and a suspending agent.

However, the inventor of the present invention has found out when preparing an oral icaritin preparation that icaritin preparations disclosed in the prior art generally have a low bioavailability. Thus, there is a need for a new oral preparation of icaritin which is suitable for large-scale industrial production and has a high bioavailability.

### Summary of the Invention

An object of the present invention is to provide a nano-micelle preparation of icaritin, the preparation having the advantage of high bioavailability.

Another object of the present invention is to provide a method for preparing the nano-micelle preparation of icaritin of the present invention.

According to one aspect of the present invention, provided is a nano-micelle preparation of icaritin, the preparation comprising: icaritin and a polymer auxiliary material.

Preferably, the preparation is an oral preparation.

Preferably, the preparation is a liquid preparation or a solid preparation.

Preferably, when the preparation is a solid preparation, the preparation comprises by weight: 1 part of icaritin and 1-20 parts of the polymer auxiliary material.

More preferably, the preparation comprises by weight: 1 part of icaritin and 6 parts of the polymer auxiliary material.

Preferably, when the preparation is a liquid preparation, the preparation comprises by weight: 1 part of icaritin, 2-20 parts of the polymer auxiliary material and 20-500 parts of water.

Preferably, when the preparation is a liquid preparation, the preparation comprises by weight: 1 part of icaritin, 6 parts of the polymer auxiliary material and 193 parts of water.

Preferably, when the preparation is a solid preparation, icaritin therein is present in an amorphous state.

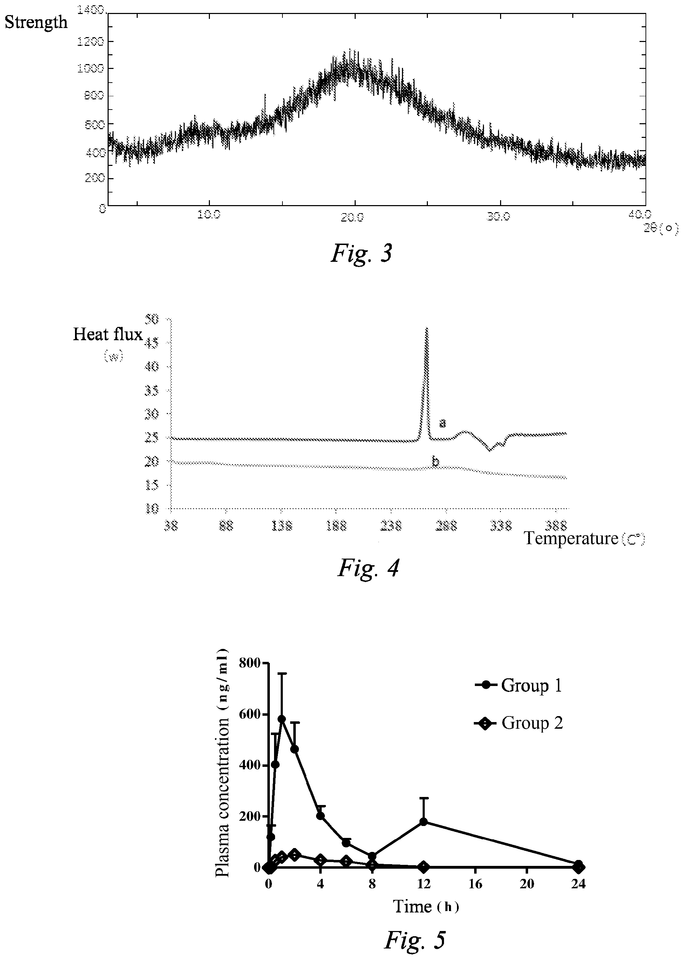

Preferably, when the nano-micelle preparation of icaritin is a solid preparation, the X-ray powder diffraction pattern is shown in Figure 3.

Preferably, the polymer auxiliary material is selected from one or more of polyvinyl caprolactam-polyvinyl acetate-polyethylene glycol graft copolymer, copovidone VA 64, polyethylene glycol 4000, sodium carboxymethyl cellulose, polyvinylpyrrolidone K25, polyvinylpyrrolidone K30, polyvinylpyrrolidone K90, poloxamer 188, poloxamer 407, tocopherol polyethylene glycol succinate and polyethylene glycol-15 hydroxystearate.

According to another aspect of the present invention, provided is a method for preparing the nano-micelle preparation of icaritin of the present invention, the method comprising the following steps:
A) firstly, dissolving icaritin in a solvent capable of dissolving icaritin to form an icaritin solution;
B) dispersing the polymer auxiliary material in a solvent capable of dissolving the auxiliary material to form a polymer solution;
C) adding the icaritin solution to the polymer solution to form a suspension;
D) centrifuging the suspension and removing a precipitate to collect a supernatant; and
E) concentrating the supernatant into a micelle concentrate.

Preferably, the method further comprises Step E' after Step E: adding one or more of a flavouring agent, a preservative and an antifoaming agent to the micelle concentrate of icaritin, and mixing same evenly to obtain a liquid nano-micelle preparation of icaritin.

Preferably, the method further comprises the steps of:
F) adding a freeze-drying auxiliary material into the micelle concentrate or the nano-micelle preparation of icaritin to freeze-dry same into a freeze-dried tablet or a freeze-dried powder;
preferably, the freeze-dried powder obtained by means of freeze-drying is crushed to prepare a granule, a tablet or a capsule, and the prepared nano-micelle preparation of icaritin is a solid preparation.

Preferably, the freeze-drying auxiliary material is selected from one or more of mannose, dextran, lactose, sucrose, glucose, glycine, trehalose and polyvinylpyrrolidone 10K, and the freeze-drying auxiliary material is 1-50% of the total mass of the micelle concentrate or the liquid nano-micelle preparation of icaritin.

More preferably, the freeze-drying auxiliary material is 2-20% of the total mass of the micelle concentrate or the liquid nano-micelle preparation of icaritin.

Preferably, the solvent capable of dissolving icaritin includes tetrahydrofuran, methanol, ethanol, acetone, N,N-dimethylformamide, or an alkaline aqueous solution, the alkaline aqueous solution including sodium hydroxide, potassium hydroxide, calcium hydroxide or an aqueous solution of sodium carbonate.

Preferably, the solvent capable of dissolving the polymer auxiliary material includes petroleum ether, n-hexane, water, hydrochloric acid, sulphuric acid, nitric acid, sulphurous acid or an aqueous solution of phosphoric acid.

Preferably, the flavouring agent comprises a sweetener and an aromatic, the sweetener is a non-sugar sweetener and accounts for 0.01-1% of the mass of the liquid nano-micelle preparation of icaritin, and the aromatic accounts for 0.01-5% of the mass of the liquid nano-micelle preparation of icaritin.

Preferably, the preservative accounts for 0.01-1% of the mass of the liquid nano-micelle preparation of icaritin and the antifoaming agent accounts for 0.01-1% of the mass of the liquid nano-micelle preparation of icaritin.

Preferably, in Step C), icaritin is added to the polymer solution with stirring at a speed of 20-2000 rpm/min; and in Step D), the suspension is centrifuged at a speed of 1,000-20,000 rpm/min.

Preferably, in Step E), the concentration of the concentrated solution after concentration is 5-50 mg icaritin/mL.

Preferably, a temperature value at which lyophilization is preformed ranges from -10°C to -80°C.

Preferably, the non-sugar sweetener is selected from one or more of sucralose, stevioside, steviol, mannitol, lactitol, maltitol, alitame, aspartame, saccharin, saccharin sodium, saccharin calcium, acesulfame potassium and thaumatin.

Preferably, the aromatic is selected from one or more of cherry-, lemon-, lime-, orange-, tangerine-, citrus-, mint-, strawberry-, caramel-, peach-, raspberry-, grapefruit-, vanilla-, cream-, chocolate- and grape-flavoured aromatics.

Preferably, the preservative is selected from one or more of paraben esters, chlorobutanol, propionic acid and sorbate.

Preferably, the antifoaming agent is selected from one or more of mineral oil, alcohol, fatty acid, fatty acid ester, amide, phosphate ester, silicone, polyether and polyether-modified polysiloxane antifoaming agents.

Beneficially, the present invention provides the nano-micelle preparation of icaritin and the preparation method therefor. The micelle in the nano-micelle preparation of icaritin of the present invention has a nano-sized particle size and is present in an amorphous state, leading to a high bioavailability. Compared with an oral preparation containing an icaritin corn oil, there is a 14.9-fold increase in bioavailability and a nearly 9.5-fold increase in the maximum plasma concentration in the animals administered with the nano-micelle preparation.

### Brief Description of the Drawings

Figure 1 shows a scanning electron microscope atlas of the nano-micelle of icaritin of the present invention.
Figure 2 shows a particle size distribution diagram of the nano-micelle solution of icaritin of the present invention.
Figure 3 shows an X-ray powder diffraction pattern for the freeze-dried powder of the nano-micelle of icaritin of Example 2 of the present invention.
Figure 4 shows a map for the differential scanning calorimetry for the freeze-dried powder of the solid nano-micelle of icaritin of Example 2 of the present invention.
Figure 5 shows plasma concentration-time curves of the nano-micelle oral solution of icaritin and an icaritin oil-dispersed soft capsule in a beagle dog.

### Detailed Description of Embodiments

Unless otherwise specified, the term "nano-micelle" herein is a complex with an external hydrophilic polar part and an internal hydrophobic part, which complex is jointly formed by icaritin and a polymer auxiliary material as observed under a scanning electron microscope, when the icaritin preparation is a liquid (including the state in which water is added to the freeze-dried preparation). Under the scanning electron microscope, a nano-micelle can be spherical, lamellar or rod-like, the diameter or width of which ranges from tens of nanometers to a few hundred nanometers.

Unless otherwise specified, the term "solid preparation" herein comprises both the nano-micelle freeze-dried preparation of the present invention and the solid preparation obtained by drying the liquid nano-micelle preparation of the present invention.

Unless otherwise specified, the term "amorphous state" herein means that icaritin is present in an amorphous state in the solid nano-micelle preparation of icaritin (including a dried liquid preparation), as measured by using a Cu-Ka ray in which the X-ray powder diffraction shows a dispersion peak in the 2θ range of 0-40°.

Unless otherwise specified, "polyvinyl caprolactam-polyvinyl acetate-polyethylene glycol graft copolymer" herein is purchased from BASF under trade name Soluplus.

Unless otherwise specified, the term "copovidone VA 64" herein is a watersoluble copolymer synthesised from N-vinyl-2-pyrrolidone and vinyl acetate at a ratio of 60 : 40. Copovidone VA 64 herein is purchased from BASF under trade names of Kollidon VA 64 and Kollidon VA 64 Fine.

Unless otherwise specified, the term "polyethylene glycol 4000" herein is a high molecular polymer formed by the polycondensation of ethylene oxide and water, with a molecular formula expressed as HO(CH₂CH₂O)ₙH, where n represents an average number of oxyethylene groups and approximates 4000 in polyethylene glycol 4000.

Unless otherwise specified, the term "sodium carboxymethyl cellulose" herein is purchased from Ashland, with a molecular weight of 250,000-720,000.

Unless otherwise specified, the term "polyvinylpyrrolidone K25" herein refers to a polymer of vinylpyrrolidone, where the K value represents an average molecular weight range of the polymer of vinylpyrrolidone. The K value is a characteristic value associated with relative viscosity of an aqueous PVP solution, and the viscosity is a physical quantity related to the molecular weight of the polymer. Therefore, the K value can be used to characterize the average molecular weight of PVP. Generally, the greater the K value, the greater the viscosity, leading to a stronger bonding property. Polyvinylpyrrolidone K25 herein is purchased from Ashland under trade name Plasdone PVP K25.

Unless otherwise specified, the term "polyvinylpyrrolidone K30" herein is defined with reference to polyvinylpyrrolidone K25, and polyvinylpyrrolidone K30 is purchased from Ashland under trade name Plasdone PVP K30.

Unless otherwise specified, the term "polyvinylpyrrolidone K90" herein is defined with reference to polyvinylpyrrolidone K25, and polyvinylpyrrolidone K90 herein is purchased from Ashland under trade name Plasdone PVP K90.

Unless otherwise specified, the term "poloxamer 188" herein, also known as Pluronic F68, refers to a PEO-PPO-PEO triblock copolymer consisting of polyoxyethylene, polyoxypropylene and polyoxyethylene, with an average molecular weight of 7680-9510, wherein an ethenoxy unit accounts for 75-85%, and an oxypropylene unit accounts for 25-30%. The trade name of poloxamer 188 is Kolliphor P188.

Unless otherwise specified, the term "poloxamer 407" herein is composed of about 70% ethylene oxide and 30% propylene oxide, with the average molecular weight of 9840-14600 under trade name Kolliphor P407.

Unless otherwise specified, the terms "tocopherol polyethylene glycol succinate and 2-hydroxyethyl 12-hydroxy-18 acid ester" herein are referred to as TPGS for short, with a cas number of 9002-96-4 under trade name Kolliphor TPGS.

Unless otherwise specified, the term "polyethylene glycol-15 hydroxystearate" herein corresponds to "Solutol HS15" in English, with a cas number of 70142-34-6.

Unless otherwise specified, the term "flavouring agent" herein refers to a pharmaceutical auxiliary material for improving or masking an undesirable odor, strong bitterness or other unpleasant taste like a pungent taste to such an extent that it is difficult for a user to detect.

Unless otherwise specified, the term "sweetener" herein refers to a pharmaceutical additive that imparts a sweet taste to the preparation of the present invention.

Unless otherwise specified, the term "aromatic" herein refers to a pharmaceutical additive that imparts an odor to the preparation of the present invention.

Unless otherwise specified, the term "simethicone" herein is purchased from Guangzhou Biaomei Pharmaceutical auxiliary material Co., Ltd., with a CAS number of 8050-81-5, which is used as an antifoaming agent in the present invention.

Unless otherwise specified, the term "Span 85" herein, also known as "Sorbitan Trioleate", is purchased from Xi'an Jinxiang Pharmaceutical Auxiliary Material Co., Ltd. with a trade number of 18091874298, which is used as an antifoaming agent in the present invention.

Unless otherwise specified, the term "freeze-drying auxiliary material" herein, also referred to as a freeze-drying proppant or a freeze-drying auxiliary material, serves as an auxiliary material that maintains the state, stability and good resolubility of a preparation after the preparation is freeze-dried.

Unless otherwise specified, the term "capsule" herein refers to a solid preparation in which a drug or a suitable auxiliary material is filled in a hollow hard capsule or sealed in a soft capsule or compressed into a tablet. A capsule can be categorized into a hard capsule, a soft capsule (gelatin pearl), a sustained-release capsule, a controlled-release capsule, an enteric-coated capsule or a tablet, mainly for oral use.

Unless otherwise specified, the term "AUCₗₐₛₜ" herein denotes the area enclosed by a plasma concentration curve and a time axis from the time of administration to the last time point. "AUCₗₐₛₜ" is often referred to as "drug exposure".

Unless otherwise specified, the term "AUC_{INF}" herein denotes the area enclosed by the time axis and the plasma concentration curve from 0 to infinity.

### Example 1

### Preparation of icaritin nano-micelle oral liquid

2.4 g of icaritin raw material is dissolved in 120 mL of 0.2 mol/L aqueous sodium hydroxide solution, and 4.8 g of polyvinyl caprolactam-polyvinyl acetate-polyethylene glycol graft copolymer and 4.8 g of poloxamer 407 are dissolved in 480 mL of 0.05 mol/L aqueous hydrochloric acid solution. With stirring at a high speed of 1000 rpm/min, the sodium hydroxide solution mixed with icaritin is added to the aqueous hydrochloric acid solution of the polymers through a peristaltic pump, and then stirred for 20 minutes. The mixture is then centrifuged at 10,000 rpm/min for 20 minutes to remove the precipitate, and the supernatant is an icaritin micelle solution. The concentration of icaritin is 3.6 mg/mL. The resulting solution is ultrafiltered via a regenerated cellulose filter membrane (100 KDa) in an ultrafiltration cup to obtain an icaritin micelle solution at a higher concentration, wherein the concentration of icaritin is 30 mg/mL.

The resulting mixed icaritin solution is placed in a transparent test bottle, and a bright "path" is observed in the solution from the direction perpendicular to the incident light when a beam of light is transmitted through the solution. Therefore, Tyndall effect occurs in the mixed icaritin solution, forming a mixed icaritin micelle solution.

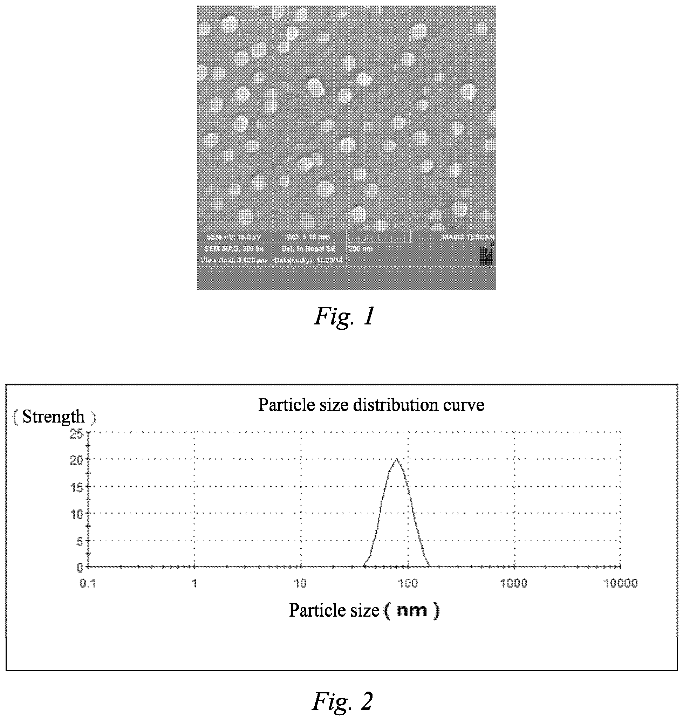

The icaritin micelle solution is examined under an electron microscope (10 KV, a scanning electron microscope), to obtain an electron microscopic atlas as shown in Figure 1. By comparison between Figure 1 and the ruler, it can be seen that an icaritin micelle particle has a nano-sized particle size distribution, with a particle size of below 200 nm.

The micelle solution obtained in Example 1 is processed with a dynamic light scattering laser particle size analyser (Malvern Master size Nano ZS) to obtain the particle size distribution as shown in Figure 2. As illustrated by the particle size distribution curve in Figure 2, icaritin micelles are in a monodisperse system with an average particle size of about 80 nm.

### Example 2

### Preparation of icaritin micelle concentrate

2 g of icaritin raw material is dissolved in 140 mL of acetone, while 20.0 g of polyethylene glycol 15 hydroxystearate and 5.0 g of tocopherol polyethylene glycol succinate are dissolved in 500 mL of purified water. The acetone solution in which icaritin is dissolved is added dropwise to a solution comprising polyethylene glycol 15 hydroxystearate and tocopherol polyethylene glycol succinate with stirring at a high speed of 1000 rpm/min through a peristaltic pump. Afterwards, the mixture is stirred for 20 minutes. Afterwards, the reaction solution is placed in a ventilated place to volatilize the acetone therein for more than 36 hours with stirring at 200 rpm/min until the acetone is completely volatilized. The remaining solution is centrifuged at 20°C and 12000 rpm/min for 20 minutes to remove the precipitate, and the supernatant is the icaritin micelle solution. The concentration of icaritin is 6 mg/mL. A regenerated cellulose filter membrane (100 Kda) is used for membrane filtration and concentration to prepare 12 mg/mL of icaritin micelle concentrate solution.

### Example 3

### Preparation of icaritin micelle concentrate

1.0 g of icaritin is dissolved in 70 mL of acetone solution, and 20.0 g of polyethylene glycol 15 hydroxystearate and 0.25 g of lauryl sodium sulphate are dissolved in 200 mL of purified water. The acetone solution in which icaritin is dissolved is added dropwise to the solution comprising polyethylene glycol 15 hydroxystearate and lauryl sodium sulphate with stirring at a high speed of 1000 rpm/min through a peristaltic pump. Afterwards, the mixture is stirred for 10 minutes. Afterwards, the reaction solution is placed in a ventilated place to volatilize the acetone therein for more than 24 hours with stirring at 300 rpm/min until the acetone is completely volatilized. The remaining solution is centrifuged at 20°C and 12000 rpm/min for 20 minutes to remove the precipitate, and the supernatant is the icaritin micelle solution. The concentration of icaritin is 5 mg/mL. A regenerated cellulose filter membrane (100 Kda) is used for membrane filtration and concentration to prepare 45 mg/mL of icaritin micelle solution.

### Example 4

### Preparation of icaritin micelle concentrate

1.2 g of icaritin raw material is dissolved in 60 mL of 0.2 mol/L aqueous sodium hydroxide solution, and 2.4 g of tocopherol polyethylene glycol succinate and 1.2 g of poloxamer 188 are dissolved in 240 mL of 0.05 mol/L aqueous hydrochloric acid solution. With stirring at a high speed of 1000 rpm/min, the sodium hydroxide solution mixed with icaritin is added to the aqueous hydrochloric acid solution of the polymers through a peristaltic pump, and then stirred for 20 minutes. Then the mixture is centrifuged at 10000 rpm/min for 20 minutes to remove the precipitate, and the supernatant is the icaritin micelle solution. The concentration of icaritin is 3.8 mg/mL. The resulting solution is ultrafiltered via a regenerated cellulose filter membrane (100 Kda) in an ultrafiltration cup to obtain the icaritin micelle concentrate solution at a higher concentration, wherein the concentration of icaritin is 20 mg/mL.

### Example 5

### Preparation of icaritin micelle concentrate

2.4 g of icaritin raw material is dissolved in 60 mL of 0.4 mol/L aqueous sodium hydroxide solution, while 9.6 g of copovidone VA 64 is dissolved in 240 mL of 0.1 mol/L aqueous hydrochloric acid solution. With stirring at a high speed of 1000 rpm/min, the sodium hydroxide solution mixed with icaritin is added to the aqueous hydrochloric acid solution of the polymers through a peristaltic pump, and then stirred for 20 minutes. Then the mixture is centrifuged at 5000 rpm/min for 20 minutes to remove the precipitate, and the supernatant is the icaritin micelle solution. The concentration of icaritin is 7.8 mg/mL. The resulting solution is ultrafiltered via a polyphenylene ether sulfone filter membrane (100 Kda) in the ultrafiltration cup to obtain the icaritin micelle concentrate solution at a higher concentration, wherein the concentration of icaritin is 28 mg/mL.

### Example 6

### Preparation of icaritin micelle oral liquid

An icaritin micelle concentrate is prepared according to the method of Examples 1 to 3. Other ingredients are added according to the prescription composition as shown in the following table:

| Prescription composition | Prescription 1 | Prescription 2 | Prescription 3 |
|---|---|---|---|
| Icaritin micelle concentrate | 30 mg/ml | 12 mg/ml | 45 mg/ml |
| Simethicone | 0.20 g | / | / |
| Strawberry- flavoured fragrance | / | 0.40 g | 0.40 g |
| Vanilla-flavoured fragrance | 0.80 g | / | 0.80 g |
| Sucralose | 0.30 g | 0.10 g | 0.45 g |
| Methyl paraben | 0.45 g | 0.45 g | 0.45 g |
| Disodium hydrogen phosphate-sodium monohydrogen phosphate buffer solution | pH = 6.8 | pH = 6.8 | pH = 6.8 |
| Total volume | 400 ml | 400 ml | 400 ml |

While constant stirring, an anti-foaming agent-simethicone, strawberry-flavoured fragrance, vanilla-flavoured fragrance, sucralose, and paraben are sequentially added to the solution of Examples 1 to 3 with reference to prescribed dosage and ratio. Then the pH value of the solution is adjusted to 6.8 by adding a disodium hydrogen phosphate-sodium dihydrogen phosphate buffer solution. After being stirred thoroughly and uniformly, and filtered, the prepared solution is placed into an amber glass bottle and sealed for storage.

### Example 7

### Preparation of icaritin micelle oral liquid

An icaritin micelle concentrate is prepared according to the method of Examples 4 to 5. Other ingredients are added according to the prescription composition as shown in the following table:

| Prescription composition | Prescription 4 | Prescription 5 |
|---|---|---|
| Icaritin micelle concentrate | 20 mg/ml | 28 mg/ml |
| Simethicone | 0.10 g | / |
| Span 85 | / | 0.20 g |
| Strawberry- flavoured fragrance | 0.40 g | / |
| Vanilla-flavoured fragrance | 0.40 g | 0.80 g |
| Sucralose | 0.20 g | 0.30 g |
| Methyl paraben | 0.45 g | 0.30 g |
| Propylparaben | / | 0.20 g |
| Disodium hydrogen phosphate-sodium dihydrogen phosphate buffer solution | pH = 6.0 | pH = 6.0 |
| Total volume | 400 ml | 400 ml |

While constant stirring, an anti-foaming agent-simethicone, Span 85, strawberry-flavoured fragrance, vanilla-flavoured fragrance, sucralose, methyl paraben and propyl paraben are sequentially added to the solution of Examples 1 to 3 with reference to prescribed dosage and ratio. Then the pH value of the solution is adjusted to 6.0 by adding a disodium hydrogen phosphate-sodium dihydrogen phosphate buffer solution.

After being stirred thoroughly and uniformly, and filtered, the prepared solution is placed into an amber glass bottle and sealed for storage.

### Example 8

Icaritin and 5% w/w sucrose are added to the micelle concentrate of Example 2, mixed and stirred uniformly, and divided into aliquots. The aliquots are placed in 10 mL glass bottles (3 mL/bottle) and subjected to freeze-drying for 48 hours to form a freeze-dried powder of the icaritin nano-micelle. The freeze-dried powder is subjected to X-ray powder diffraction pattern identification to obtain a pattern as shown in Figure 3. It can be seen from Figure 3 that a dispersion peak appears on the X-ray powder diffraction pattern. A differential scanning calorimetry test is performed. Figure 4 shows that curve b and curve a are differential scanning calorimetry curves of the freeze-dried powder of the icaritin nano-micelle of this example and the anhydrate crystal form of icaritin, respectively. Curve b is a flat curve, presenting no heat change over the entire temperature range between 38°C and 388°C and curve a is the curve of the anhydrate crystal form of icaritin, presenting an obvious melting endothermic peak at about 260°C. It can be seen therefrom that, the freeze-dried powder of the solid nano-micelle of icaritin prepared according to the method of this example does not comprise an icaritin crystal, but an amorphous state of icaritin.

### Example 9

Preparation of icaritin tablet. Lactose as a diluent, microcrystalline cellulose as a disintegrant, croscarmellose sodium as a binder, and hydroxy propyl cellulose and magnesium stearate as lubricants are added to the freeze-dried powder prepared according to Example 8.

| Prescription composition | Prescription 1 | Prescription 2 |
|---|---|---|
| Icaritin freeze-dried powder (calculated by icaritin) | 100 mg | 50 mg |
| Lactose | 100 mg | 50 mg |
| Microcrystalline cellulose | 20 mg | 10 mg |
| Croscarmellose sodium | 20 mg | 10 mg |
| Hydroxy propyl cellulose | 4 mg | 2 mg |
| Magnesium stearate | 8 mg | 4 mg |
| Silicon dioxide | 8 mg | 4 mg |

The icaritin freeze-dried powder is taken and crushed and filtered via a sieve to make particle size D₉₀ ≤ 75 µm for later use; a prescribed dosage of the freeze-dried powder of icaritin (calculated by icaritin) is mixed well with lactose, microcrystalline cellulose, 1/3 prescribed dosage of croscarmellose sodium and 1/2 prescribed dosage of magnesium stearate, then subjected to non-slurry pelletizing and processed by a granulate machine with a 30-mesh sievie. The remaining 2/3 prescribed dosage of croscarmellose sodium, hydroxy propyl cellulose, the remaining 1/2 prescribed dosage of magnesium stearate, and silicon dioxide are added and mixed; After tabletting with a tablet machine, the mixture is packed for storage.

### Example 10 Preparation of icaritin freeze-dried tablet

The icaritin micelle concentrate is prepared according to the method in Example 1. Other ingredients are added according to the prescription composition as shown in the following table:

| Prescription composition | Prescription |
|---|---|
| Icaritin micelle concentrate (calculated by icaritin) | 3 ml, 20 mg/mL |
| Simethicone | 0.03 g |
| Vanilla-flavoured fragrance | 0.15 g |
| Stevioside | 0.03 g |
| Sucrose | 0.1 g |
| Disodium hydrogen phosphate-sodium dihydrogen phosphate buffer solution | pH = 6.8 |

Simethicone, a fragrance, stevioside, and sucrose are added to the icaritin micelle concentrate in sequence according to the prescribed dosage, and stirred well before the disodium hydrogen phosphate-sodium dihydrogen phosphate buffer solution is added to adjust the pH value to 6.8.

The solution is filled into a double-aluminium packaging mould, and transferred to a freeze-drying device for freeze-drying to prepare a freeze-dried tablet.

### Example 11

### Hard capsule of icaritin nano-micelle freeze-dried powder

The icaritin micelle freeze-dried powder is prepared by lyophilization according to the method of Example 8, and according to HPLC, the drug loading capacity thereof is 14%.

The freeze-dried powder is filtered via a 40-mesh sieve. Afterwards, 714 mg of the freeze-dried powder is filled into No. 1 hard capsule and taken as an icaritin capsule, wherein the capsule comprises 100 mg of icaritin.

### Comparative Example

### Preparation of icaritin soft capsule

The anhydrate crystal form of icaritin with a purity of more than 97% is ground and filtered through a 170-mesh sieve. For preparing icaritin raw material, refer to 200710099025.1, and for preparing the anhydrate crystal form thereof, refer to 201410185323.2. A detection is performed, and the finely ground icaritin has a particle size of less than 90 µm. Then 0.4 g of beeswax and 15 g of corn oil are heated to 70°C for dissolving and evenly mixing; icaritin is added to the solution comprising corn oil and beeswax to prepare an oil-soluble substance. The oil-soluble substance is added to a soft capsule to make an icaritin oil-soluble soft capsule, wherein each icaritin soft capsule comprises 100 mg of icaritin.

### Example 12

### In vivo pharmacokinetic experiment in beagle dogs

Male beagle dogs (2-3 years old, approximately 8-10 kg) are purchased from Beijing Marshall Biotechnology Co. Ltd., and placed in a room at 23 ± 2°C and 55 ± 10% humidity, with a 12-hour day/night interval, while water and food are available. After being deprived of food and water for 24 hours, the beagle dogs are administered the icaritin nano-micelle oral liquid of Example 1 and the icaritin soft capsule of the Comparative Example, both at a dosage of 20 mg icaritin/Kg body weight. Blood samples are collected from peripheral veins at 0, 0.167, 0.5, 1, 2, 4, 6, 8, 12, and 24 hours, and then centrifuged at 4000 rpm/min for 10 minutes at 2-8°C to obtain plasma. Then the plasma is stored at -75 ± 15°C. The icaritin nano-micelle oral liquid is for Group 1, and the icaritin soft capsule for Group 2.

This experiment aims to evaluate whether the oral bioavailability of icaritin are enhanced in Group 1 compared with that in Group 2. Pharmacokinetics studies are carried out in the beagle dogs, as their pharmacokinetic parameters differ significantly from those in a mouse, but are close to those in a human being. Four beagle dogs are used for Group 1 while six are used for Group 2. The pharmacokinetic parameters and comparative values are listed in Table 1. Figure 5 shows the plasma concentration curve over the entire experiment. After an oral administration, the maximum concentration Cₘₐₓ of icaritin is 595 ± 350 ng/mL in the fasting state in Group 1, which is 9.5 times higher than that in Group 2 (p < 0.05). In Group 1, AUCₗₐₛₜ is 3592 ± 2267 h^{∗}ng/mL, which is 14.9 times greater than that in Group 2 (p < 0.05). These results reveal that the nano-micelle preparation state in Group 1 can significantly increase the absorption of icaritin. Group 1 shows a higher icaritin solubility and permeability over Group 2, leading to a significant increase in Cₘₐₓ and AUCₗₐₛₜ of icaritin.

The pharmacokinetic parameters in Group 1 and Group 2 in which the beagle dogs have been dosed are shown below.

**Table 1**

| PK parameters | Group 1 (n = 4) | Group 2 (n = 6) |
|---|---|---|
| Tₘₐₓ(h) | 1.25 ± 0.50 | 1.42 ± 0.66 |
| Cₘₐₓ (ng/mL) | 595 ± 350 | 62.7 ± 14.3 |
| T_{half-life} (h) | NA | 2.04 ± 0.32 |
| AUCₗₐₛₜ (hr^{∗}ng/mL) | 3592 ±2267 | 241 ± 283 |
| AUC_{INF} (hr^{∗}ng/mL) | NA | 245 ± 293 |

| | | |
|---|---|---|
| NA: Fail to report a value. | | |

## Claims

1. A nano-micelle preparation of icaritin, wherein the preparation comprises: icaritin and a polymer auxiliary material.

2. The preparation according to claim 1, wherein the preparation is an oral preparation.

3. The preparation according to claim 2, wherein the preparation is a liquid preparation or a solid preparation.

4. The preparation according to claim 1 or 3, wherein when the preparation is a solid preparation, the preparation comprises by weight: 1 part of icaritin and 1-20 parts of the polymer auxiliary material; and preferably, the preparation comprises by weight: 1 part of icaritin and 6 parts of the polymer auxiliary material.

5. The preparation according to claim 1 or 3, wherein when the preparation is a liquid preparation, the preparation comprises by weight: 1 part of icaritin, 2-20 parts of the polymer auxiliary material and 20-500 parts of water; and preferably, when the preparation is a liquid preparation, the preparation comprises by weight: 1 part of icaritin, 6 parts of the polymer auxiliary material and 193 parts of water.

6. The preparation according to claim 1 or 3, wherein when the preparation is a solid preparation, icaritin therein is present in an amorphous state.

7. The preparation according to claim 6, wherein when the nano-micelle preparation of icaritin is a solid preparation, the X-ray powder diffraction pattern is shown in Figure 3.

8. The preparation according to claim 1, wherein the polymer auxiliary material is selected from one or more of polyvinyl caprolactam-polyvinyl acetate-polyethylene glycol graft copolymer, copovidone VA64, polyethylene glycol 4000, sodium carboxymethyl cellulose, polyvinylpyrrolidone K25, polyvinylpyrrolidone K30, polyvinylpyrrolidone K90, poloxamer 188, poloxamer 407, tocopherol polyethylene glycol succinate and polyethylene glycol-15 hydroxystearate.

9. A method for preparing the nano-micelle of icaritin according to any one of claims 1-8, wherein the method comprises the following steps:
A) firstly, dissolving icaritin in a solvent capable of dissolving icaritin to form an icaritin solution;
B) dispersing the polymer auxiliary material in a solvent capable of dissolving the auxiliary material to form a polymer solution;
C) adding the icaritin solution to the polymer solution to form a suspension;
D) centrifuging the suspension and removing a precipitate to collect a supernatant; and
E) concentrating the supernatant into a micelle concentrate.

10. The method according to claim 9, wherein the method further comprises Step E' after Step E: adding one or more of a flavouring agent, a preservative and an antifoaming agent to the micelle concentrate of icaritin, and mixing same evenly to obtain a liquid nano-micelle preparation of icaritin.

11. The method according to claim 9 or 10, wherein the method comprises the following steps:
F) adding a freeze-drying auxiliary material into the micelle concentrate or the liquid nano-micelle preparation of icaritin to freeze-dry same into a freeze-dried tablet or a freeze-dried powder.

12. The method according to claim 11, wherein the freeze-dried powder obtained by means of freeze-drying is crushed to prepare a granule, a tablet or a capsule, and the prepared nano-micelle preparation of icaritin is a solid preparation.

13. The method according to claim 11, wherein the freeze-drying auxiliary material is selected from one or more of mannose, dextran, lactose, sucrose, glucose, glycine, trehalose and polyvinylpyrrolidone 10K, and the freeze-drying auxiliary material is 1-50% of the total mass of the micelle concentrate or the liquid nano-micelle preparation of icaritin; and preferably, the freeze-drying auxiliary material is 2-20% of the total mass of the micelle concentrate or the liquid nano-micelle preparation of icaritin.

14. The method according to claim 9, wherein the solvent capable of dissolving icaritin includes tetrahydrofuran, methanol, ethanol, acetone, N,N-dimethylformamide, or an alkaline aqueous solution, the alkaline aqueous solution including sodium hydroxide, potassium hydroxide, calcium hydroxide or an aqueous solution of sodium carbonate.

15. The method according to claim 9, wherein the solvent capable of dissolving the polymer auxiliary material includes petroleum ether, n-hexane, water, hydrochloric acid, sulphuric acid, nitric acid, sulphurous acid or an aqueous solution of phosphoric acid.

16. The method according to claim 10, wherein the flavouring agent comprises a sweetener and an aromatic, the sweetener is a non-sugar sweetener and accounts for 0.01-1% of the mass of the liquid nano-micelle preparation of icaritin, and the aromatic accounts for 0.01-5% of the mass of the liquid nano-micelle preparation of icaritin.

17. The method according to claim 10, wherein the preservative accounts for 0.01-1% of the mass of the liquid nano-micelle preparation of icaritin and the antifoaming agent accounts for 0.01-1% of the mass of the liquid nano-micelle preparation of icaritin.

18. The method according to claim 9, wherein in Step C), the icaritin solution is added to the polymer solution with stirring at a speed of 20-2000 rpm/min; and in Step D), the suspension is centrifuged at a speed of 1,000-20,000 rpm/min.

19. The method according to claim 9, wherein in Step E), the concentration of the concentrated solution after concentration is 5-50 mg icaritin/mL.
